# EUROPEAN PATENT APPLICATION

(11) **EP 3 210 608 A1**
(43) Date of publication of application: **30.08.2017**
(21) Application number: 15853138.4
(22) Date of filing: 23.10.2015
(51) Int. Cl.: A61K 31/4709, A61K 9/20, A61P 31/04

(54) **SOLID PHARMACEUTICAL COMPOSITION**

(30) Priority: 23.10.2014 JP 2014216515
(71) Applicant: Kyorin Pharmaceutical Co., Ltd., Tokyo 101-8311 (JP)
(72) Inventor: UCHIDA, Hiroshi, Tochigi 329-0114 (JP); HANADA, Masataka, Tochigi 329-0114 (JP)
(74) Representative: Vossius & Partner Patentanwälte Rechtsanwälte mbB
(86) International application number: PCT/JP2015/005344
(87) International publication number: WO 2016/063544

(57) **Abstract**

[Problem] Provided is a pharmaceutical composition which suppresses gelation of the 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride and which suppresses elution delay.

[Solution] This solid pharmaceutical composition is obtained by using crystals (B-form crystals) of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride, having peaks at 9.4 and 17.7 degrees (±0.2 degrees for each angle) as 2θ diffraction angles in X-ray powder diffraction using CuKα emission.

## Description

### Technical Field

The present invention relates to a solid pharmaceutical composition containing 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride.

### Background Art

As 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylicacidhydrochloride, two kinds of crystals (A-form and B-form crystals) have been known (Patent Literature 1).

Some pharmaceutically active components which cause gelation under a certain condition have been known (Patent Literatures 2 to 8 and Non-Patent Literatures 1 and 2). In general, when a solid dosage form is orally administrated, the solid dosage form readily disintegrates in the gastrointestinal tract to dissolve a pharmaceutically active component, whereby the pharmaceutically active component is absorbed into the body. However, when a solid dosage form containing a pharmaceutically active component which causes gelation is administrated, there arises such a problem that gelation of the pharmaceutically active component delays the disintegration of the solid dosage form, and delays the dissolution of the pharmaceutically active component.

As conventional techniques of improving delayed disintegration due to gelation, a method of adding cyclodextrin to suppress gel formation or secure the water permeability of a gel layer (Non-Patent Literatures 1 and 2), a method of adding a disintegrant (Non-Patent Literature 1), a method of adding a silicic acid or a silicate (Patent Literatures 2 to 4), a method of making a drug finer and causing the drug to be adsorbed to a carrier (Patent Literature 5), a method in which a film coating is rapidly broken to disintegrate a drug-containing core before gelation (Patent Literature 6), a method using an acidic or basic additive (Patent Literature 7), a method of achieving a form of molecular dispersion such as dispersion of a drug in a polymer (Patent Literature 8), and a method of adding a sugar alcohol (Patent Literatures 9 to 11) have been known.

### Citation List

### Patent Literature

Patent Literature 1: WO2013/069297
Patent Literature 2: JP2006-298811
Patent Literature 3: WO2006/030826
Patent Literature 4: JP2002-505290
Patent Literature 5: JP2004-522782
Patent Literature 6: JP1987-123118
Patent Literature 7: WO2006/059716
Patent Literature 8: JP2002-530338
Patent Literature 9: WO2013/145749
Patent Literature 10: WO2013/1457450
Patent Literature 11: JP2013-43834

### Non-Patent Literature

Non-Patent Literature 1: Journal of Pharmaceutical Science and Technology, Japan, Vol. 55, No. 3 (1995), pp. 175-182
Non-Patent Literature 2 : PharmTech Japan, vol. 17, No. 4 (2001), pp. 87-100 (619-632)

### Summary of Invention

### Technical Problem

The present invention provides a novel solid pharmaceutical composition which can suppress delayed release of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride, and a method for producing the same.

### Solution to Problem

The inventors have found that when in a solid pharmaceutical composition containing 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride, a crystal (B-form crystal) of a hydrate of the hydrochloride having peaks at 9.4 degrees and 17.7 degrees (±0.2 degrees for each angle) as 2θ diffraction angles in X-ray powder diffraction is used, delayed release can be suppressed. The present invention has thus been completed.

The summary of the present invention is as follows:
[1] A solid pharmaceutical composition containing a crystal of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride having peaks at 9.4 degrees and 17.7 degrees (±0.2 degrees for each angle) as 2θ diffraction angles in X-ray powder diffraction using CuKα radiation, wherein the solid pharmaceutical composition is obtained by compression-molding.
[2] The solid pharmaceutical composition according to [1], further containing a disintegrant.
[3] The solid pharmaceutical composition according to [2], containing a granulated substance containing the crystal, and the disintegrant.
[4] Aproductionmethodof the solidpharmaceutical composition according to any one of [1] to [3], the method including:
   obtaining a raw material for compression-molding containing the crystal; and
   compression-molding the obtained raw material for compression-molding, wherein
   the solid pharmaceutical composition is a tablet, and
   a dissolution rate in a first fluid for dissolution test is 60% or more 30 minutes after initiation of a dissolution test which is performed in accordance with a method of dissolution test in the Japanese Pharmacopoeia Sixteenth Edition.
[5] The production method according to [4], wherein
   the solid pharmaceutical composition is the solid pharmaceutical composition according to [2] or [3], and
   the raw material for compression-molding is obtained by mixing the crystal with the disintegrant, and the obtained raw material for compression-molding is subjected to direct compression molding.
[6] The production method according to [4], wherein
   the solid pharmaceutical composition is the solid pharmaceutical composition according to [2] or [3], and
   the production method includes obtaining a granulated substance containing the crystal, and obtaining the raw material for compression-molding by mixing the obtained granulated substance with the disintegrant.
[7] A method for suppressing delayed dissolution of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride contained in a tablet, the method including:
   allowing the tablet to contain a crystal of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride having peaks at 9.4 degrees and 17.7 degrees (±0.2 degrees for each angle) as 2θ diffraction angles in X-ray powder diffraction using CuKα radiation as the 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride.
[8] A solid pharmaceutical composition containing:
   a granular substance containing a crystal of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride having peaks at 9.4 degrees and 17.7 degrees (±0.2 degrees for each angle) as 2θ diffraction angles in X-ray powder diffraction using CuKα radiation; and
   a disintegrant.
[9] The solid pharmaceutical composition according to [2] or [3], wherein the disintegrant is low-substituted hydroxypropyl cellulose.
[10] The production method according to [5] or [6], wherein the disintegrant is low-substituted hydroxypropyl cellulose.
[11] The solid pharmaceutical composition according to [8], wherein the disintegrant is low-substituted hydroxypropyl cellulose.
[12] The solid pharmaceutical composition according to [1] or [2], which is produced by a direct compression method.
[13] The solid pharmaceutical composition according to any one of [1] to [3], which is produced by a dry granulation method.
[14] The production method according to [6], wherein the granulated substance is produced by a dry granulation method.
[15] The solid pharmaceutical composition according to [8], wherein the granulated substance is produced by a dry granulation method.

### Advantageous Effects of Invention

The present invention can provide a novel solid pharmaceutical composition which can suppress delayed release of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride, and a production method of the same.

### Brief Description of Drawings

FIG. 1 is an X-ray powder diffraction pattern of a crystal (A-form crystal) of an anhydride of 7-[(3S,4S)-3-{(cyclopropylamino)methyl}-4-fluoropyrrolidine-1-yl}-6-fluoro-1-(2-fluoroethyl)-8-methoxy-4-oxo-1,4-dihydroquin oline-3-carboxylic acid hydrochloride.
FIG. 2 is a table describing peaks of which the relative intensity is 0.7 or more when the intensity of a peak at a 2θ of 4.9 degrees in the diffraction pattern shown in FIG. 1 is taken as 100.
FIG. 3 is an X-ray powder diffraction pattern of a crystal (B-form crystal) of a hydrate of 7-[(3S,4S)-3-{(cyclopropylamino)methyl}-4-fluoropyrrolidine-1-yl}-6-fluoro-1-(2-fluoroethyl)-8-methoxy-4-oxo-1,4-dihydroquin oline-3-carboxylic acid hydrochloride.
FIG. 4 is a table describing peaks of which the relative intensity is 0.7 or more when the intensity of a peak at a 2θ of 4.8 degrees in the diffraction pattern shown in FIG. 3 is taken as 100.
FIG. 5 shows results of a dissolution test of tablets obtained immediately after production in Example 1 and Comparative Example 1 (dissolution media: first fluid for the dissolution test).
FIG. 6 shows results of a dissolution test of tablets obtained immediately after production in Example 2 and Comparative Example 2 (dissolution media: first fluid for the dissolution test). Description of Embodiments

Hereinafter, one of embodiments of the present invention will be described in detail.

This embodiment relates to a solid pharmaceutical composition containing a crystal (hereinafter also referred to as B-form crystal) of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride having peaks at 9.4 degrees and 17.7 degrees (±0.2 degrees for each angle) as 2θ diffraction angles in X-ray powder diffraction using CuKα radiation.

The solid pharmaceutical composition containing 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride is likely to delay dissolution. Herein, an A-form crystal (anhydride) and a B-form crystal (hydrate) are known as forms of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride. In general,itisknown that an anhydride is more rapidly dissolved than a hydrate. However, in a case of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride, it has been found that more excellent dissolution is exerted by use of a B-form crystal which is a hydrate. That is, when a B-form crystal having peaks at 4.8 degrees, 9.4 degrees, 10.3 degrees, 13.6 degrees, 14.2 degrees, 17.7 degrees, 21.5 degrees, 22.8 degrees, 25.8 degrees, and 27.0 degrees as 2θ diffraction angles in X-ray powder diffraction using CuKα radiation is contained in the solid pharmaceutical composition, delayed dissolution can be suppressed. Characteristic peaks of the B-form crystal are confirmed at 4.8 degrees, 9.4 degrees, 17.7 degrees, 22.8degrees, 25.8degrees, and 27.0 degrees as 2θ diffraction angles. In particular, characteristic peaks are confirmed at 9.4 degrees and 17.7 degrees.

Herein, X-ray powder diffraction can be performed using RINT2200 manufactured by Rigaku Denki Co., Ltd. Copper radiation (CuKα radiation) is used as radiation. A measurement condition may include a tube current of 36 mA, a tube voltage of 40 kV, a divergence slit of 1 degree, a scattering slit of 1 degree, a receiving slit of 0.15 mm, a scanning range of 1 to 40 degrees (2θ), and a scanning speed of 2 degrees (2θ) per minute.

The dissolution rate of the solid pharmaceutical composition containing the B-form crystal of this embodiment is higher than that of another solid pharmaceutical composition containing the hydrochloride of the compound described above. In a preferred aspect, when a dissolution test is performed in accordance with a method of dissolution test in the Japanese Pharmacopoeia Sixteenth Edition, the dissolution rate in a first fluid for dissolution test 30 minutes after initiation of the dissolution test is 60% or more. In the solid pharmaceutical composition of this embodiment, the dissolution rate in the first fluid for dissolution test 30 minutes after initiation of the dissolution test is more preferably 70% or more, and further preferably 75% or more.

The solid pharmaceutical composition herein means a pharmaceutical composition constituted by a solid component to be contained. The solid pharmaceutical composition of this embodiment may be an oral composition. Specifically, the solid pharmaceutical composition of this embodiment may be an oral composition such as a tablet, a granule (fine granule), a capsule, and a powder. A tablet is preferred. The ratio of each component contained in the solid pharmaceutical composition of this embodiment is not particularly limited, and can be appropriately set according to the dosage form or the like by those skilled in the art.

For example, when the solid pharmaceutical composition of this embodiment is prepared as a tablet, the preferable content of the crystal (B-form crystal) of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride may be 10% by mass or more and 70% by mass or less relative to the weight of a whole uncoated tablet. The content thereof is more preferably 20% by mass or more and 60% by mass or less, particularly preferably 30% by mass or more and 50% by mass or less, and further preferably 35% by mass or more and 45% by mass or less.

The uncoated tablet herein means a tablet into which a raw material is pressed and which is in a state before forming a coating thereon.

The solid pharmaceutical composition of this embodiment may be produced by a procedure including compression-molding a raw material for compression-molding containing the B-form crystal described above. The solid pharmaceutical composition of this embodiment can be produced in accordance with a general method, and the production method can be appropriately selected by those skilled in the art. Examples of the general method may include a method including a step of producing a granulated substance and a direct compression method. When the solid pharmaceutical composition of this embodiment is produced through the step of producing a granulated substance, the granulation may be performed in accordance with a dry granulation method or a wet granulation method. The granulated substance (granular substance) herein means a granular molded body obtained by hardening a solid component.

The "dry granulation method" described herein is a method in which a raw material powder is compression-molded, crushed, and classified into particles having appropriate size to obtain a granulated substance. In the dry granulation method, water is not used. Therefore, the crystal (B-form crystal) of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride is not transferred into another crystal or an amorphous material, and delayed release is suppressed. Accordingly, the dry granulation method is preferred.

The "wet granulation method" described herein is a method in which water or a binder solution is added dropwise to or sprayed on a raw material powder, and granulated, and the obtained granulated substance in a wet state is dried. Examples of the wet granulation method may include an extruding granulation method, a fluidized bed granulation method, an tumbling granulation method, a disintegrating granulation method, a spray drying granulation method, and a agitating granulation method. When the wet granulation method is used, granulation can be performed using an organic solvent other than water. Therefore, drying at low temperature is possible and crystalline transition from the B-form crystal to another crystal or an amorphous material can be suppressed. Accordingly, granulation using an organic solvent other than water is preferred.

When the wet granulation method is used, a part of the B-form crystal is subjected to crystalline transition, and the dissolution rate may be decreased. Therefore, it is preferable that the obtained granulated substance be mixed with a disintegrant and the obtained mixture be pressed into a tablet.

The tablet produced as described above contains the granulated substance (granular substance) and the disintegrant, and the disintegrant is disposed on a periphery of the granulated substance. The disintegrant promotes rapid permeation of water into the inside of the tablet. Therefore, water is easy to reach the B-form crystal contained in the granulated substance, and the dissolution rate can be improved.

In addition to the B-form crystal, the granulated substance (granular substance) may contain the disintegrant. In addition to the disintegrant, another additive may be contained outside the granulated substance. When the solid pharmaceutical composition of this embodiment contains a plurality of granulated substances (granular substances), one part of the granulated substances may be exposed to a surface of an uncoated tablet.

Further, the solid pharmaceutical composition of this embodiment may be produced by a direct compression method without the step of granulation. The "direct compression method" is a method in which a mixture obtained by adding a needed additive such as an excipient to a raw material containing an active ingredient is molded by compression without a granulation treatment (direct compression-molding) to obtain an uncoated tablet. In the direct compression method, water is not used like the dry granulation method. Therefore, the B-form crystal is unlikely to be transferred into another crystal or an amorphous material, and delayed release is suppressed. Therefore, the direct compression method is preferred.

In addition to the crystal (B-form crystal) of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride, the solid pharmaceutical composition of this embodiment may contain an additive such as an excipient, a disintegrant, a binder, and a lubricant. The usable additive is not particularly limited as long as it can be used in production of a pharmaceutical dosage form. For example, additives described in "Japanese Pharmaceutical Excipients Directory (International Pharmaceutical Excipients Council Japan, YAKUJI NIPPO, LTD. (2007)) can be appropriately used.

The excipient described herein includes a "cellulosic excipient" and a "non-cellulosic excipient."

The "cellulosic excipient" described herein is an excipient containing cellulose or a derivative thereof as a component. Example of the cellulosic excipient may include microcrystalline cellulose , carmellose, carmellose calcium, carmellose sodium, croscarmellose sodium, carboxymethyl cellulose calcium, and low substituted hydroxypropyl cellulose. Among these, it is preferable that the cellulosic excipient that can be contained in the solid pharmaceutical composition of this embodiment be microcrystalline cellulose since the hardness of a molded tablet can be made high.

The "non-cellulosic excipient" described herein is an excipient having no cellulose skeleton in its structure. Examples thereof may include monosaccharides such as glucose and fructose, disaccharides such as lactose, sucrose, maltose, trehalose, and maltose, starches such as corn starch, and sugar alcohols including monosaccharide alcohols such as mannitol, sorbitol, xylitol, and erythritol, and disaccharide alcohols such as isomalt, maltitol, and lactitol.

The solid pharmaceutical composition of this embodiment may contain one or two or more of the above-described excipients. Also, the solid pharmaceutical composition of this embodiment may contain a combination of the cellulosic excipient and the non-cellulosic excipient. For example, microcrystalline cellulose and lactose may be used in combination.

A preferable content of the excipient is as follows.
1) Case where the solid pharmaceutical composition of this embodiment contains a granulated substance and is obtained by compression-molding a mixture of the granulated substance with another component and the excipient exists outside the granulated substance
   The content of the excipient existing outside the granulated substance is preferably 0.1 parts by mass or more and 0.8 parts by mass or less relative to 1 part by mass of the granulated substance. The content thereof is more preferably 0.2 parts by mass or more and 0.7 parts by mass or less, and particularly preferably 0.4 parts by mass or more and 0.6 parts by mass or less.
2) Case where the solid pharmaceutical composition of this embodiment contains a granulated substance and is obtained by compression-molding a mixture of the granulated substance with another component and the excipient exists in the granulated substance
   The content of the excipient in the granulated substance is preferably 5% by mass or more and 50% by mass or less relative to the amount of the whole granulated substance. The content thereof is more preferably 10% by mass or more and 40% by mass or less, particularly preferably 15% by mass or more and 30% by mass or less, and further preferably 20% by mass or more and 30% by mass or less.
3) Case of using direct compression method
   When a direct compression method is used, the content of the excipient is preferably 10% by mass or more and 80% by mass or less relative to the amount of the whole solid pharmaceutical composition (the amount of the whole uncoated tablet when a coating is formed on the solid pharmaceutical composition). The content thereof is more preferably 20% by mass or more and 70% by mass or less, particularly preferably 30% by mass or more and 60% by mass or less, and further preferably 40% by mass or more and 50% by mass or less.

The disintegrant is an additive of imparting disintegrant properties to the solid pharmaceutical composition in water or a gastric juice, and is classified into a swelling disintegrant and a wicking disintegrant. The swelling disintegrant is preferably used since a composition having higher hardness and lower friability is obtained.

The swelling disintegrant is a disintegrant which absorbs water to swell, causing disintegration of an oral solid dosage form. Examples thereof may include hydroxypropyl starch, low-substituted hydroxypropyl cellulose, sodium carboxymethyl starch, croscarmellose, croscarmellose sodium, and crospovidone. Low-substituted hydroxypropyl cellulose is more preferred.

The wicking disintegrant is a disintegrant which causes disintegration of an oral solid dosage form by a capillary phenomenon. The wicking disintegrant absorbs water through a void to decrease an interparticle bonding force in the oral solid dosage form, thereby disintegrating the oral solid dosage form. Examples of the wicking disintegrant may include carmellose, carmellose sodium, microcrystalline cellulose and carboxymethylcellulose sodium, cellulose acetate phthalate, wheat starch, rice starch, corn starch, potato starch, pregelatinized starch, partly pregelatinized starch, and microcrystalline cellulose. Carmellose is more preferred.

It is the most preferable that the solid pharmaceutical composition of this embodiment contain low-substituted hydroxypropyl cellulose as the disintegrant.

Herein, low-substituted hydroxypropyl cellulose means hydroxypropyl cellulose of which the content of a hydroxypropoxyl group is 5.0 to 16.0% by weight. The content of a hydroxypropoxyl group can be determined by a method described in a section of "low-substituted hydroxypropyl cellulose" in the Japanese Pharmacopoeia Sixteenth Edition.

In terms of enhancing the disintegration properties of the solid pharmaceutical composition, it is preferable that the solid pharmaceutical composition be a mixture of the disintegrant with the granulated substance containing the crystal (B-form crystal) of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride. In the mixture, it is more preferable that the granulated substance be substantially uniformly dispersed.

The content of the disintegrant is preferably 2% by mass or more and 30% by mass or less relative to the amount of the whole solid pharmaceutical composition (the amount of the whole uncoated tablet when a coating is formed on the solid pharmaceutical composition). The content thereof is more preferably 5% by mass or more and 20% by mass or less, particularly preferably 5% by mass or more and 15% by mass or less, and further preferably 8% by mass or more and 12% by mass or less.

The binder is an additive used to impart a bonding force to a mixture of a pharmaceutical powder and facilitate compression molding. Examples thereof may include starch (soluble), microcrystalline cellulose, hydroxypropyl cellulose, carmellose, polyvinyl alcohol, hypromellose, dextrin, and povidone. Hydroxypropyl cellulose is more preferred.

The content of the binder is preferably 0.1% by mass or more and 10% by mass or less relative to the amount of the whole solid pharmaceutical composition (the amount of the whole uncoated tablet when a coating is formed on the solid pharmaceutical composition). The content thereof is more preferably 0.5% by mass or more and 5.0% by mass or less, particularly preferably 1.0% by mass or more and 3.0% by mass or less, and further preferably 1.5% by mass or more and 2.5% by mass or less.

The content of the binder is preferably 0.1% by mass or more and 10% by mass or less relative to the amount of the whole granulated substance. The content thereof is more preferably 1.0% by mass or more and 7.0% by mass or less, particularly preferably 2.0% by mass or more and 5.0% by mass or less, and further preferably 3.0% by mass or more and 4.0% by mass or less.

Hereinafter, the content of the solid pharmaceutical composition of this embodiment will be described more specifically with reference to one example of a production method of producing the solid pharmaceutical composition of this embodiment as a tablet, and the scope of the present invention is not limited to the example.

### (General Production Method 1)

1. The following A component, and if necessary, the following B component (excipient) or the following C component (disintegrant) are mixed. To a powder obtained by the mixing, a D component (lubricant) may be further added.
   A component: crystal (B-form crystal) of hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride
   B component: one or two or more kinds of excipients selected from the group consisting of cellulosic excipients such as microcrystalline cellulose, carmellose, carmellose calcium, carmellose sodium, croscarmellose sodium, carboxymethyl cellulose calcium, and low-substituted hydroxypropyl cellulose, monosaccharides such as glucose and fructose, disaccharides such as lactose, sucrose, maltose, trehalose, and maltose, starches such as corn starch, and sugar alcohols such as monosaccharide alcohols such as mannitol, sorbitol, xylitol, and erythritol, and disaccharide alcohols such as isomalt, maltitol, and lactitol
   C component: one or two or more kinds of disintegrants selected from the group consisting of hydroxypropyl starch, low-substituted hydroxypropyl cellulose, sodium carboxymethyl starch, croscarmellose, croscarmellose sodium, crospovidone, carmellose, carmellose sodium, microcrystalline cellulose and carboxymethylcellulose sodium, cellulose acetate phthalate, wheat starch, rice starch, corn starch, potato starch, pregelatinized starch, partly pregelatinized starch, and microcrystalline cellulose
   D component: one or two or more kinds of lubricants selected from the group consisting of stearic acid, a stearate salt (salt of metal such as aluminum, potassium, sodium, calcium, and magnesium), and sodium laurylsulfate
2. The resulting mixture (raw material for compression-molding) is pressed into a tablet by a tableting machine to obtain a tablet (uncoated tablet). After the pressing into a tablet, the resulting uncoated tablet maybe coated with a coating agent such as hypromellose and Kollicoat IR.

### (General Production Method 2)

1. The A component, and if necessary, the B component (excipient), the C component (disintegrant), or an E component (binder) are mixed. To a powder obtained by the mixing, the D component (lubricant) may be further added.
   E component: one or two or more kinds of binders selected from the group consisting of starch (soluble), microcrystalline cellulose, hydroxypropyl cellulose, carmellose, polyvinyl alcohol, hypromellose, dextrin, and povidone
2. For example, granulation is performed in accordance with a dry granulation method. Specifically, the resulting mixture is compression-molded by a compression molding device such as ROLLER COMPACTOR or a tableting machine (slug machine), and then crushed and subjected to size adjustment by a particle sizing device such as ROLL GRANULATOR or a sieve, to obtain a granulated substance.
   To the resulting granulated substance, the B component (excipient), the C component (disintegrant), or the D component (lubricant) may be further added.
3. A raw material for compression-molding which is the resulting granulated substance or a mixture of the granulated substance with an additive is pressed into a tablet by a tableting machine, to obtain a tablet (uncoated tablet). After the pressing into a tablet, the resulting uncoated tablet may be coated with a coating agent such as hypromellose and Kollicoat IR.

### (General Production Method 3)

1. The A component, and if necessary, the B component (excipient), the C component (disintegrant), or the E component (binder) are mixed. To a powder obtained by the mixing, the D component (lubricant) may be further added.
2. Granulation is performed in accordance with, for example, a wet granulation method. Specifically, the resulting mixture is granulated using water or an organic solvent, and dried by a dryer, to obtain a granulated substance.

To the obtained granulated substance, the B component (excipient), the C component (disintegrant), or the D component (lubricant) may be further added. 3. A raw material for compression-molding which is the resulting granulated substance or a mixture of the granulated substance with an additive is pressed into a tablet by a tableting machine, to obtain a tablet (uncoated tablet). After the pressing into a tablet, the resulting uncoated tablet may be coated with a coating agent such as hypromellose and Kollicoat IR.

### [Examples]

Hereinafter, the present invention will be described more in detail with reference to Examples, but the scope of the present invention is not restricted by these Examples.

In the following Examples, an NMR spectrum was measured by JEOL JNM-EX400 nuclear magnetic resonance spectrometer, using tetramethylsilane (TMS) as an internal standard. A MS spectrum was measured by JEOL JMS-T100LP and JMS-SX102A mass spectrometers. Elementary analysis was performed by YANACO CHN CORDERMT-6 elemental analyzer.

### (Reference Example 1)

### Bis(acetato-O)-[6,7-difluoro-1-(2-fluoroethyl)-8-methoxy-4-oxo-1,4-dihydroquinoline-3-carboxylate-O³,O⁴] boron

103 g (1.67 mol) of boric acid (for formation of a catalyst) was added to 21.4 L (225 mol) of acetic anhydride under a nitrogen atmosphere, and the mixture was heated and stirred at 70.0 to 76. 9°C for 30 minutes (at a stirring rate of 69.5 rpm). The mixture was cooled to an inner temperature of 24.6°C, 1.01 kg (16.3 mol) of first additional boric acid was added, and the mixture was stirred at 24.6 to 27.4°C for 30 minutes. 1.01 kg (16.3mol) of second additional boric acid was added, and the mixture was stirred at 24.7 to 27.5°C for 30 minutes. 1.01 kg (16.3 mol) of third additional boric acid was added, and the mixture was stirred at 24.7 to 27.7°C for 30 minutes. 1.01 kg (16.3 mol) of fourth additional boric acid was added, and the mixture was stirred at 25.4 to 29.4°C for 30 minutes. In addition, the mixture was stirred at 50.0 to 56.9°C for 30 minutes, to prepare a boric acid triacetate preparation liquid. To the preparation liquid, 5.50 kg (16.7 mol) of 6,7-difluoro-1-(2-fluoroethyl)-8-methoxy-4-oxo-1,4-dihydroquin oline-3-carboxylic acid ethyl ester was added, and the resulting mixed liquid was stirred at 54.7 to 56.9°C for 3 hours. The mixed liquid was cooled to 30.0°C, and allowed to stand at room temperature overnight. The mixed liquid was heated to 58.6°C to dissolve the deposited compound, and 16.5 L of acetone was added to the mixed liquid to obtain a reaction liquid (a).

A mixed liquid of 193 L of water and 33.7 L (555 mol) of ammonia water (28%) was cooled to -0.6°C under a nitrogen atmosphere. To the mixed liquid, the reaction liquid (a) was added, and the vessel for the reaction liguid (a) was washed with 11.0 L of acetone. The mixture was cooled to 15.0°C, and stirred at 4.3 to 15.0°C for 1 hour. The deposited crystal was collected by filtration, and washed with 55.0 L of water to obtain 14.1 kg of crude wet crystal. The crude wet crystal was dried under reduced pressure at a setting temperature of 65.0°C for about 22 hours to obtain 6.93 kg of crude crystal (yield: 96.7%).

To the resulting crude crystal, 34.7 L of acetone was added under a nitrogen atmosphere, and the mixture was heated (at hot water setting temperature of 57.0°C) to dissolve the crude crystal. During the heating, 69.3 L of diisopropyl ether was added dropwise (dropwise addition amount: 12.0 L) until crystallization. After confirmation of crystallization, the mixture was stirred at 48.3 to 51.7°C for 15 minutes, the rest of diisopropyl ether was added dropwise, and the mixture was stirred at 45.8 to 49. 7°C for 15 minutes. The mixture was cooled to 15.0°C, and stirred at 6.5 to 15.0°C for 30 minutes. The deposited crystal was collected by filtration, and washed with 6.93 L of acetone and 13.9 L of diisopropyl ether, to obtain 7.41 kg of wet crystal. The resulting wet crystal was dried under reduced pressure at a setting temperature of 65.0°C for about 20 hours, to obtain 6.47 kg of bis(acetato-O)-[6,7-difluoro-1-(2-fluoroethyl)-8-methoxy-4-oxo -1,4-dihydroquinoline-3-carboxylate-O³,O⁴] boron (yield: 90.3%).

Elemental Analysis (%) : as C₁₇H₁₅BF₃NO₈
Calculated value: C, 47.58; H, 3.52; N, 3.26.
Measured value: C, 47.41; H, 3.41; N, 3.20.
¹H-NMR (CDCl₃, 400 MHz) δ: 2.04 (6H, s), 4.21 (3H, d, J = 2.9 Hz), 4.88 (2H, dt, J = 47.0, 4.4 Hz), 5.21 (2H, dt, J = 24.9, 3.9 Hz), 8.17 (1H, t, J = 8.8 Hz), 9.10 (1H, s).
ESI MS(positive) m/z: 430 (M+H)⁺.

### (Reference Example 2)

### Production of 7-[(3S,4S)-3-{(cyclopropylamino)methyl}-4-fluoropyrrolidine-1-yl]-6-fluoro-1-(2-fluoroethyl)-8-methoxy-4-oxo-1,4-dihydroquin oline-3-carboxylic acid hydrochloride

A mixed liquid of 3.56 kg (15.4 mol) of (3R,4S)-3-cyclopropylaminomethyl-4-fluoropyrrolidine, 11.7 L (84.2 mol) of triethylamine, and 30.0 L of dimethylsulfoxide was stirred at 23.0 to 26.3°C for 15 minutes under a nitrogen atmosphere. To the mixed liquid, 6.00 kg (14.0 mol) of bis(acetato-O)-[6,7-difluoro-1-(2-fluoroethyl)-8-methoxy-4-oxo -1,4-dihydroquinoline-3-carboxylate-O³,O⁴] boron was added at 23.0 to 26.3°C to obtain a reaction liquid. The reaction liquid was stirred at 23.7 to 26.3°C for 2 hours. To the reaction liquid, 120 L of ethyl acetate was added, 120 L of water was further added, a solution of 960 g (amount corresponding to 2 mol/L) of sodium hydroxide and 12.0 L of water was added, and the mixture was stirred for 5 minutes. After that, an aqueous layer was separated. To the aqueous layer, 120 L of ethyl acetate was added, and the mixture was stirred for 5 minutes. After that, an ethyl acetate layer was separated. The ethyl acetate layer was collected, 120 L of water was added, and the mixture was stirred for 5 minutes and allowed to stand. After that, an aqueous layer was discarded. The ethyl acetate layer was distilled off under reduced pressure. The resulting residue was dissolved in 60.0 L of 2-propanol, and allowed to stand at room temperature overnight. To the solution, a solution of 5.24 L (62.9 mol) of hydrochloric acid and 26.2 L (amount corresponding to 2 mol/L) of water was added, and the mixture was stirred at 28.2 to 30.0°C for 30 minutes. The mixture was heated at an external temperature of 55.0°C. After dissolution (dissolution was confirmed at 47.1°C), the mixture was cooled, resulting in crystallization. The mixture was stirred at 39.9 to 41.0°C for 30 minutes, cooled (guide: to 20.0°C at a setting temperature of 7.0°C, and to 20.0°C or lower at -10.0°C), and stirred at 2.2 to 10.0°C for 1 hour. The deposited crystal was collected by filtration, and washed with 60 L of 2-propanol, to obtain 9.57 kg of crude wet crystal of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride.

### (Reference Example 3)

### Method for producing crystal of anhydride of 7-[(3S,4S)-3-{(cyclopropylamino)methyl}-4-fluoropyrrolidine-1-yl]-6-fluoro-1-(2-fluoroethyl)-8-methoxy-4-oxo-1,4-dihydroquin oline-3-carboxylic acid hydrochloride (hereinafter referred to as A-form crystal or Compound 1)

9.57 kg of crude wet crystal of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride was added to a mixed liquid of 60 L of ethanol and 10.8 L of purified water, and dissolved by heating. This dissolved solution was passed through a filter, and washed with a mixed liquid of 24.0 L of ethanol and 1.20 L of purified water. Dissolution was confirmed, and 96.0 L of heated ethanol (99.5) was added at 71.2 to 72.6°C. The dissolved solution was cooled (hot water setting temperature: 60.0°C). After confirmation of crystallization (crystallization temperature: 61.5°C), the solution was stirred at 59.4 to 61.5°C for 30 minutes. The solution was stepwisely cooled (to 50.0°C at a hot water setting temperature of 40.0°C, to 40.0°C at a hot water setting temperature of 30.0°C, to 30.0°C at a hot water setting temperature of 20.0°C, to 20.0°C at a setting temperature of 7.0°C, and to 15.0°C at a setting temperature of -10.0°C, and allowed to stand), and stirred at 4.8 to 10.0°C for 1 hour. The deposited crystal was collected by filtration, and washed with 30.0 L of ethanol, to obtain 5.25 kg of wet crystal of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride. The resulting wet crystal was dried under reduced pressure at a setting temperature of 50.0°C for about 13 hours to obtain 4.83 kg of Compound 1 (yield: 72.6%).

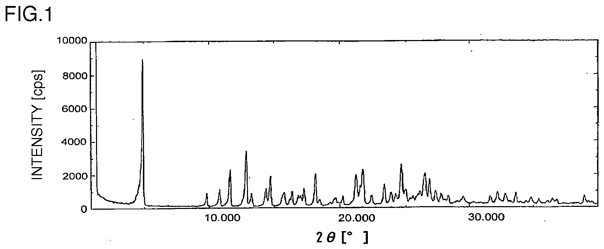

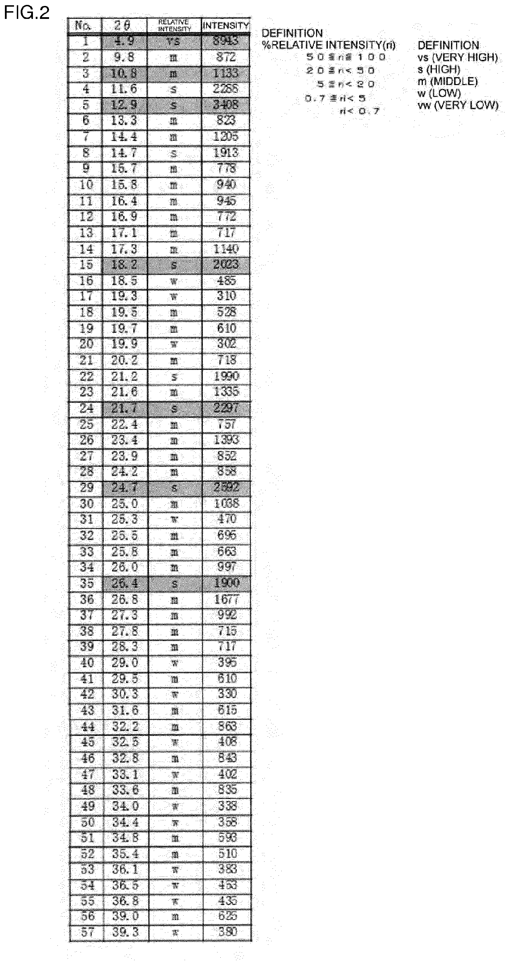

FIG. 1 shows a result of X-ray powder diffraction of Compound 1 based on WO2013/069297. FIG. 2 shows a table describing relative intensities of peaks contained in the diffraction pattern shown in FIG. 1.

X-ray powder diffraction was performed using RINT2200 manufactured by Rigaku Denki Co., Ltd. Copper radiation was used as radiation. As a measurement condition, a tube current of 36 mA, a tube voltage of 40 kV, a divergence slit of 1 degree, a scattering slit of 1 degree, a receiving slit of 0.15 mm, a scanning range of 1 to 40 degrees (2θ), and a scanning speed of 2 degrees (2θ) per minute were used. For a crystal in Reference Example 4 described below, measurement was performed under the same condition.

As understood from FIGs. 1 and 2, peaks are found at 4.9 degrees, 9.8 degrees, 10.8 degrees, 12.9 degrees, 14.7 degrees, 18.2 degrees, 21.7 degrees, 23.4 degrees, 24.7 degrees, and 26.4 degrees, and characteristic peaks are confirmed at 4.9 degrees, 10.8 degrees, 12.9 degrees, 18.2 degrees, 21.7 degrees, 24.7 degrees, and 26.4 degrees. In particular, characteristic peaks are confirmed at 10.8 degrees, 12.9 degrees, and 24.7 degrees.
Elemental Analysis Value (%): as C₂₁H₂₄F₃N₃O₄HCl
Calculated value: C, 53.00; H, 5.30; N, 8.83.
Measured value: C, 53.04; H, 5.18; N, 8.83.
¹H NMR (DMSO-d₆, 400 MHz) δ (ppm): 0.77-0.81 (2H, m), 0.95 - 1.06 (2H, m), 2. 80 - 2.90 (2H, m), 3.21 - 3.24 (1H, m), 3.35 - 3.39 (1H, m), 3.57 (3H, s), 3.65 - 3.78 (3H, m), 4.13 (1H, dd, J = 41.8, 13.1 Hz), 4.64 - 4.97 (3H, m), 5.14 (1H, dd, J = 32.7, 15.6 Hz), 5.50 (1H, d, J = 53.7 Hz), 7.80 (1H, d, J = 13.7 Hz), 8.86 (1H, s), 9.44 (2H, brs), 15.11 (1H, brs).
ESI MS(positive) m/z: 440 (M+H)⁺.

### (Reference Example 4)

### Crystal (B-form crystal) of hydrate of 7-[(3S,4S)-3-{(cyclopropylamino)methyl}-4-fluoropyrrolidine-1-yl]-6-fluoro-1-(2-fluoroethyl)-8-methoxy-4-oxo-1,4-dihydroquin oline-3-carboxylic acid hydrochloride (hereinafter also referred to as Compound 2)

30.0 g (63.0 mmol) of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride obtained in Reference Example 2 was added to a mixed solvent of 600 mL of 2-propanol and 90.0 mL of water, and dissolved by heating (inner temperature: 72°C). The dissolved solution was cooled, and crystallization was confirmed (inner temperature: 49°C). After that, the dissolved solution was stirred at a temperature near the crystallization temperature for 5 minutes (inner temperature: 48 to 49°C). The dissolved solution was heated until the inner temperature was increased from the crystallization temperature by about 10°C, and then stirred at this temperature for 30 minutes (inner temperature: 48 to 60°C). The dissolved solution was gradually cooled (cooled at about 1°C/min), and stirred at 10°C or lower for 1 hour (inner temperature: 2 to 10°C). The deposited crystal was collected by filtration, and washed with a mixed solvent of 143 mL of 2-propanol and 7.5 mL of water to obtain 34.5 g of a crystal (B-form crystal) of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride as a white powder.

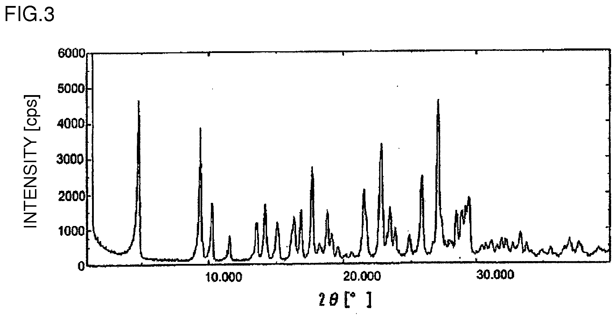

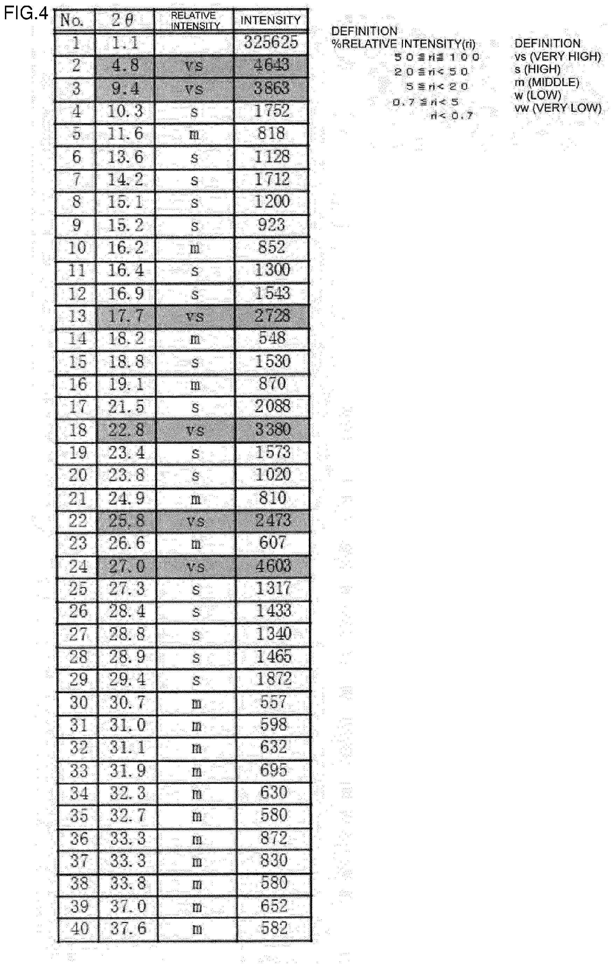

FIG. 3 shows a result of X-ray powder diffraction of Compound 2 based on WO2013/069297. FIG. 4 shows a table describing relative intensities of peaks contained in the diffraction pattern shown in FIG. 3.

As understood from FIGs. 3 and 4, peaks are found at 4.8 degrees, 9.4 degrees, 10.3 degrees, 13.6 degrees, 14.2 degrees, 17.7 degrees, 21.5 degrees, 22.8 degrees, 25.8 degrees, and 27.0 degrees, and characteristic peaks are confirmed at 4.8 degrees, 9.4 degrees, 17.7 degrees, 22.8 degrees, 25.8 degrees, and 27.0 degrees. In particular, characteristic peaks are confirmed at 9.4 degrees and 17.7 degrees.
¹H NMR (DMSO-d₆, 400 MHz) δ (ppm) : 0 .77 - 0.81 (2H, m), 0.98 - 1.00 (2H, m), 2.79 - 2.93 (2H, m), 3.22 (1H, dd, J = 8.4, 12.2 Hz), 3.58 (3H, s), 3.65 - 3.81 (3H, m), 4.13 (1H, dd, J = 13.2, 42.1 Hz), 4.81 - 4.97 (2H, m), 5.15 (1H, dd, J = 15.7, 32.8 Hz), 5.55 (1H, d, J = 53.8 Hz), 7.79 (1H, dd, J = 2.4, 13.2 Hz), 8.85 (s, 1H), 9.56 (2H, brs), 15.07 (1H, brs)

### (Example 1)

In accordance with prescription described in Table 1, Compound 2 (B-form crystal), lactose, microcrystalline cellulose, and low-substituted hydroxypropyl cellulose were mixed using a pestle and a mortar. To the mixture, magnesium stearate was added, and the mixture was mixed using a pestle and a mortar. The mixture was pressed into a tablet using a tableting machine (HT-AP-18SS-II, manufactured by Hata Tekkosho Co., Ltd., mortar with a diameter of 7.5 mm, punch with a R plane having a radius of curvature of 9 mm) so that the mass was 190 mg and the thickness of a tablet was 3.9 mm. An aqueous coating was formed using a mixture of hypromellose, titanium oxide, polyethylene glycol 400, and yellow ferric oxide by Hicoater (trade name) (HCT-MINI manufactured by Freund Corporation).

### (Comparative Example 1)

The same operation as that in Example 1 was performed except that the operation is performed in accordance with prescription described in Table 1. Compound 1 (A-form crystal) was used in place of Compound 2 (B-form crystal).

**[Table 1]**

| PRESCRIPTION | EXAMPLE 1 | COMPARATIVE EXAMPLE 1 |
|---|---|---|
| COMPOUND 2 (B-FORM CRYSTAL) | 81.2 | - |
| COMPOUND 1 (A-FORM CRYSTAL) | - | 81.2 |
| MICROCRYSTALLINE CELLULOSE | 22.1 | 22.1 |
| LACTOSE | 66.4 | 66.4 |
| LOW-SUBSTITUTED HYDROXYPROPYL CELLULOSE | 19 | 19 |
| MAGNESIUM STEARATE | 1.3 | 1.3 |
| SUBTOTAL (mg) | 190 | 190 |
| HYPROMELLOSE | 3.6 | 3.6 |
| TITANIUM OXIDE | 1.96 | 1.96 |
| POLYETHYLENE GLYCOL 400 | 0.36 | 0.36 |
| YELLOW FERRIC OXIDE | 0.08 | 0.08 |
| TOTAL (mg) | 196 | 196 |

### (Test Example 1) Dissolution Test (First fluid for dissolution test)

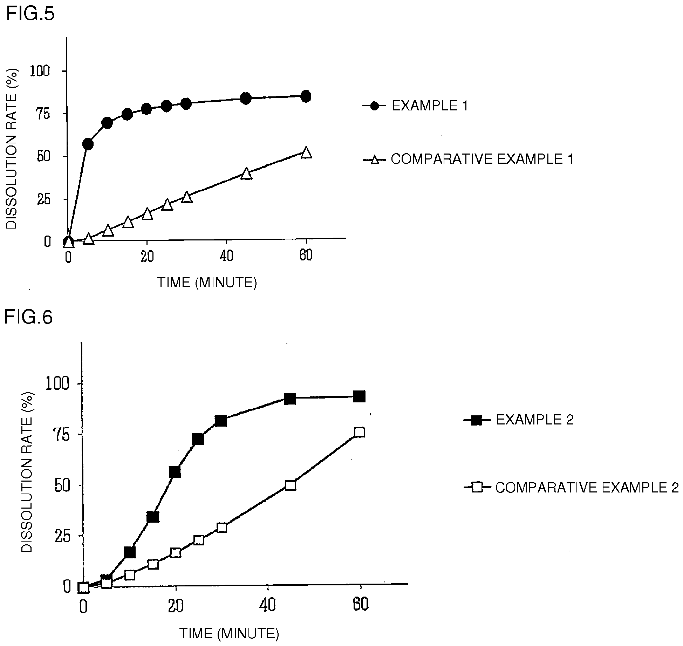

In order to evaluate each of the compositions (tablets) in Example 1 and Comparative Example 1, a dissolution test was performed in accordance with dissolution apparatus 2 (paddle method) in the Japanese Pharmacopoeia Sixteenth Edition. Detailed conditions of the dissolution test are as follows. The results of the dissolution test are shown in FIG. 5.
Paddle rotation speed: 50 rpm
Temperature of dissolution media: 37°C
Dissolution media : 900 mL of first fluid for dissolution test in the Japanese Pharmacopoeia Sixteenth Edition

As seen from the results of FIG. 5, the dissolution rate of the tablet containing Compound 2 (B-form crystal) in the first fluid for dissolution test is higher than that of the tablet containing Compound 1 (A-form crystal). When Compound 2 (B-form crystal) is used, the dissolution rate in the first fluid for dissolution test reaches 80% or more 30 minutes after the initiation of the dissolution test. When Compound 1 (A-form crystal) is used, the dissolution rate in the first fluid for dissolution test is at most about 25% 30 minutes after the initiation of the dissolution test.

As described above, 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride other than the B-form crystal has a property of causing gelation by contact with water. In the solid pharmaceutical composition of Example 1, the B-from crystal which is unlikely to be gelled as compared with the other hydrochloride is used. As a result, significant improvement in the dissolution rate of the hydrochloride is succeeded.

### (Example 2)

In accordance with prescription described in Table 2, Compound 2 (B-form crystal), lactose, microcrystalline cellulose, and hydroxypropyl cellulose were mixed using a pestle and a mortar. After that, to the mixture, water was added and the mixture was kneaded. The kneaded substance was granulated using a sieve with an opening of 1.4 mm, and dried by a dryer at 80°C for 60 minutes. The resulting granulated substance was passed through a sieve with an opening of 850 µm, to obtain a sieved product as main drug granules. The main drug granules, lactose, microcrystalline cellulose, and low-substituted hydroxypropyl cellulose were then mixed using a pestle and a mortar. To the mixture, magnesium stearate was added, and the mixture was mixed using a pestle and a mortar. The mixture was pressed into a tablet using a tableting machine (HT-AP-18SS-II, manufactured by Hata Tekkosho Co., Ltd., mortar with a diameter of 7.5 mm, punch with a R plane having a radius of curvature of 9 mm) so that the mass was 190 mg and the thickness of a tablet was 3.9 mm. An aqueous coating was formed using a mixture of hypromellose, titanium oxide, polyethylene glycol 400, and yellow ferric oxide by a Hicoater (trade name) (HCT-MINI manufactured by Freund Corporation).

### (Comparative Example 2)

The same operation as that in Example was performed except that the operation is performed in accordance with prescription described in Table 2 and Compound 1 (A-form crystal) was used in place of Compound 2 (B-form crystal).

**[Table 2]**

| PRESCRIPTION | EXAMPLE 2 | COMPARATIVE EXAMPLE 2 |
|---|---|---|
| COMPOUND 2 (B-FORM CRYSTAL) | 81.2 | - |
| COMPOUND 1 (A-FORM CRYSTAL) | - | 81.2 |
| MICROCRYSTALLINE CELLULOSE | 8.5 | 8.5 |
| LACTOSE | 19 | 19 |
| HYDROXYPROPYL CELLULOSE | 3.8 | 3.8 |
| SUBTOTAL (mg) | 112.5 | 112.5 |
| MICROCRYSTALLINE CELLULOSE | 17.6 | 17.6 |
| LACTOSE | 39.6 | 39.6 |
| LOW-SUBSTITUTED HYDROXYPROPYL CELLULOSE | 19 | 19 |
| MAGNESIUM STEARATE | 1.3 | 1.3 |
| SUBTOTAL (mg) | 190 | 190 |
| HYPROMELLOSE | 3.6 | 3.6 |
| TITANIUM OXIDE | 1.96 | 1.96 |
| POLYETHYLENE GLYCOL 400 | 0.36 | 0.36 |
| YELLOW FERRIC OXIDE | 0.08 | 0.08 |
| TOTAL (mg) | 196 | 196 |

### (Test Example 2) Dissolution Test (First fluid for dissolution test)

In order to evaluate each of the compositions (tablets) in Example 2 and Comparative Example 2, a dissolution test was performed in the same manner as in Test Example 1. The results of the dissolution test are shown in FIG. 6.

As seen from the results of FIG. 6, when a B-form crystal is also used for a tablet prepared by a wet granulation method, the dissolution rate in the first fluid for dissolution test reaches 80% or more 30 minutes after the initiation of the dissolution test. On the other hand, when Compound 1 (A-form crystal) is used, the dissolution rate in the first fluid for dissolution test is at most 30% or less 30 minutes after the initiation of the dissolution test (Comparative Example 2). When the B-from crystal which is unlikely to be gelled as compared with the other hydrochloride is used, the dissolution rate of the hydrochloride can be significantly improved.

### Industrial Applicability

When the crystal (B-form crystal) of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride having peaks at 9.4 degrees and 17.7 degrees (±0.2 degrees for each angle) as 2θ diffraction angles is used, a solid pharmaceutical composition which suppresses delayed dissolution of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride can be provided.

## Claims

1. A solid pharmaceutical composition comprising a crystal of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride having peaks at 9.4 degrees and 17.7 degrees (±0.2 degrees for each angle) as 2θ diffraction angles in X-ray powder diffraction using CuKα radiation, wherein the solid pharmaceutical composition is obtained by compression-molding.

2. The solid pharmaceutical composition according to claim 1, further comprising a disintegrant.

3. The solid pharmaceutical composition according to claim 2, comprising a granulated substance containing the crystal, and the disintegrant.

4. A production method of the solid pharmaceutical composition according to any one of claims 1 to 3, the method comprising:
obtaining a raw material for compression-molding containing the crystal; and
compression-molding the obtained raw material for compression-molding, wherein
the solid pharmaceutical composition is a tablet, and
a dissolution rate in a first fluid for dissolution test is 60% or more 30 minutes after initiation of a dissolution test which is performed in accordance with a method of dissolution test in the Japanese Pharmacopoeia Sixteenth Edition.

5. The production method according to claim 4, wherein
the solid pharmaceutical composition is the solid pharmaceutical composition according to claim 2 or 3, and
the raw material for compression-molding is obtained by mixing the crystal with the disintegrant, and the obtained raw material for compression-molding is subjected to direct compression molding.

6. The production method according to claim 4, wherein
the solid pharmaceutical composition is the solid pharmaceutical composition according to claim 2 or 3, and
the production method includes obtaining a granulated substance containing the crystal, and obtaining the raw material for compression-molding by mixing the obtained granulated substance with the disintegrant.

7. A method for suppressing delayed dissolution of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride contained in a tablet, the method comprising:
allowing the tablet to contain a crystal of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride having peaks at 9.4 degrees and 17.7 degrees (±0.2 degrees for each angle) as 2θ diffraction angles in X-ray powder diffraction using CuKα radiation as the 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride.

8. A solid pharmaceutical composition comprising:
a granular substance containing a crystal of a hydrate of 7-{(3S,4S)-3-[(cyclopropylamino)methyl]-4-fluoropyrrolidine-1-yl}-6-fluoro-1-(2-fluoroethyl)-8-methoxy-4-oxo-1,4-dihydroquin oline-3-carboxylic acid hydrochloride having peaks at 9.4 degrees and 17.7 degrees (±0.2 degrees for each angle) as 2θ diffraction angles in X-ray powder diffraction using CuKα radiation; and
a disintegrant.
